# EUROPEAN PATENT APPLICATION

(11) **EP 2 400 032 A1**
(43) Date of publication of application: **28.12.2011**
(21) Application number: 10167261.6
(22) Date of filing: 25.06.2010
(51) Int. Cl.: C12Q 1/04

(54) **An improved method for measuring microbial count**

(71) Applicant: Unilever N.V., 3013 AL Rotterdam (NL)
(72) Inventor: Medepalli, Srilaxmi Venkata, Whitefield Bangalore 560 066 (IN); Nayak, Kalpana Kamalakar, Whitefield Bangalore 560 066 (IN); Shah, Nimish Harshadrai, Whitefield Bangalore 560 066 (IN)
(74) Representative: Elliott, Peter William

(57) **Abstract**

This invention relates to a method of measuring microbial count on skin in general, and on hands in particular.

There is an unfulfilled need for a reproducible, reliable and robust method for isolation of microbes from hands since it has been observed that methods known and used in the past have high variability in isolating microbes. The present inventors have found that the variability of a commonly used conventional method i.e. the glove juice method can be reduced by mechanizing the massaging step, in a specific way.

## Description

### Technical Field

This invention relates to a method of measuring microbial count on skin in general, and on hands in particular. The invention has been developed primarily for measuring microbial count on hands and will be described hereinafter with reference to this application. However, it will be appreciated that the invention is not limited to this particular field of use.

### Background of the invention

Any discussion of the prior art throughout the specification should in no way be considered as an admission that such prior art is widely known or forms part of the common general knowledge in the field.

Microbial contamination of hands and the subsequent contact of the contaminated hands with drinking water and food is related to spreading of various gastro-intestinal infective diseases. It is estimated that washing hands with soap after defecation can reduce the incidence of such diseases by about 50%. Bacterial contamination of hands of health care professionals such as doctors and nurses is known to be a cause of spreading of serious infectious diseases and potentially fatal post-operative infections.

The methods of measuring microbial count on hands are not only important for academic studies on hand hygiene, but are also important in testing the efficacy of various hand hygiene products and appliances that are commercially available. In view of the importance of hand hygiene, there is a need for a robust, reproducible and reliable method for measuring microbial count on hands.

There are various well established methods for enumeration of microbes (especially bacteria) such as plate counts, most probable number (MPN), dye reduction tests and ATP bioluminisence

Methods for recovery of microbes from hands include the Inversion method, Rinsing method, Tape stripping and the Glove juice method. However, the problem in measuring microbial count on hands is that it is not easy to collect a representative sample from hands, i.e. to isolate microbes from hands in a reproducible and robust manner.

Known methods for isolation of microbes from hand include stripping with cellophane tape, swabbing small areas with glass cylinders, imprinting fingertips on agar plates, rubbing glass beads within plastic bags or test tubes, scrubbing hands in basins, etc.

The most widely accepted method for isolation of microbes from hands is the so-called "glove juice" method, which has been adopted by American Society for Testing Materials (ASTM) in various standards, i.e. standard for evaluation of surgical hand scrub formulations (E-1115-86, 1987, ASTM Standard Materials Environ. Microbiology 1: 201-204), and standard for evaluation of health care personnel hand wash formulations (E-1174-87, 1987, ASTM Standard Materials Environ. Microbiology 1: 209-212).

In the glove juice method, hands of a subject are placed in sterile gloves and a collection fluid, which includes water and electrolytes is poured into each of the gloves, with the top of the gloves held tight at the wrists to avoid spillage. The gloved hands are massaged for about a minute, followed by carefully removing the gloves from hands without spilling the contents, and the glove juice is collected and used for further measurement of microbial counts.

One of the difficulties associated with the glove juice method is the inherent variability in the step of massaging the gloved hands. Various factors that can influence the efficiency of collection include the force applied, the surface area covered, and frequency and duration of rubbing. The collection efficiency is essentially operator-dependent, and even for the same operator, collection efficiency varies with the fatigue level of operator. In particular, when glove juice is collected from a number of subjects by the same operator, a decline in collection efficiency is to be expected as the operator fatigue sets in.

A different problem is also known in which there is a high variability in the efficacy of washing hands by health care professionals, i.e., not every hand wash is efficacious in sterilizing the hand surface. To overcome this problem, hand washing machines have been proposed in which the hand is contacted with a cleaning or disinfection fluid whilst the rubbing action is provided by mechanical means. Contacting hands with such disinfection fluid kills the microbes and such methods cannot be utilized for measuring microbial counts.

Mahl (J. Clinical Microibiol., Oct. 1989) Vol 27 No 10, p2295-2299) have described a method wherein bacteria are inoculated in the fingernail region of hand and the region was scrubbed with an electric toothbrush to collect the run-off for measurement of bacteria. Although the collection efficiency is improved and the operator fatigue is eliminated, it is inconvenient and time-consuming to use a brush on the entire surface of hand. However, sensation of toothbrush bristles on bare skin of hand can be discomforting to the subjects. Further, there is a possibility of spillage whilst brushing or collecting the run-off.

From the discussion of the above mentioned art, it is clear that there is an unfulfilled need for a reproducible, reliable and robust method for isolation of microbes from hands.

Present inventors have found that the variability of the conventional glove juice method can be reduced by mechanizing the massaging step.

### Objects of the Invention

It is an object of the present invention to overcome or ameliorate at least one of the disadvantages of the prior art, or to provide a useful alternative.

It is another object of the present invention to provide a method for measuring microbial count on hands which has relatively less variability.

It is a further object of the present invention to provide a method for measuring microbial count on hands which is relatively more reliable, reproducible and robust.

### Summary of the Invention

According to the present invention there is provided an improved glove juice method for measuring microbial count on hands comprising the steps of:
i. Placing a hand of a subject in a sterile glove, prior to or after pouring a collection fluid in the glove;
ii. Contacting the gloved hand with a mechanical rubbing means for a predetermined amount of time to intimately contact hand surface with the collection fluid;
iii. Collecting the glove juice and measuring microbial count in the glove juice.

Preferably, the improved glove juice comprises the steps of releasably securing said glove at the wrist of the subject by a securing means to prevent spillage or leakage after step (i) but before step (iii).

### Brief Description of the Drawings

The invention will now be described by way of example only with reference to he accompanying drawings, in which:
- Figure 1 is one of the preferred embodiments of mechanical rubbing means according to the present invention.
- Figure 2 is an alternate preferred embodiment of mechanical rubbing means according to the present invention.

### Detailed Description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

According to the present invention there is provided an improved glove juice method for measuring microbial count on hands comprising the steps of:
i. Placing a hand of a subject in a sterile glove, prior to or after pouring a collection fluid in the glove;
ii. Contacting the gloved hand with a mechanical rubbing means for a predetermined amount of time to intimately contact hand surface with the collection fluid;
iii. Collecting the glove juice and measuring microbial count in the glove juice.

Any suitable glove can be used. Glove is preferably made of materials that do not absorb water. Suitable examples of such materials include plastic and rubber. Preferably, glove is not rigid. The glove according to the present invention may or may not have recesses to accommodate individual fingers. A plastic carry bag or grocery bag is preferably used as a glove.

It is preferred to use a sterile glove. The gloves can be sterilized by any suitable method capable of substantially reducing microbes, preferably eliminating microbes. Preferably, autoclaving is used for sterilization of gloves. Alternatively, pre-sterilized medical gloves or food-handler's gloves can be used.

Preferably the glove is releasably secured at the wrist of the subject using securing means. A string or a thread that can be tied around the glove at the wrist can be used as securing means. Alternatively, a rubber band can be used as a securing means.

Collection fluid preferably comprises water. It is preferred that the collection fluid further comprises a surfactant, preferably a non-ionic surfactant. The collection fluid may be buffered at a pH between 4 to 10, preferably between 5 to 9, more preferably between 6 to 8. A preferred mechanical rubbing means, according to the present invention, is a rubbing means capable of being set in motion with predetermined speed and/or periodicity. Preferably the motion is cyclic, i.e. to and fro, or clockwise and anticlockwise. Preferably, mechanical rubbing means are powered by battery, or electricity.

Predetermined contact time is between 2-300 seconds, preferably 5-200 seconds, more preferably 10-100 seconds.

According to a preferred aspect, the rubbing means include at least one rubbing member. Preferably, the rubbing member is a flat sheet. Preferably, the sheet is capable of a reciprocal or rotary motion within the plane of the said sheet. When the motion is reciprocal, the average linear speed is preferably between 0.1-2 m/s, preferably 0.3-1.2 m/s or more preferably between 0.5-1 m/s and the no of cycles is preferably between 2-200, preferably 10-120, more preferably between 30-100 cycles per second. When the motion is rotary, the angular velocity is preferably between 1-200, preferably 10-150, more preferably between 30-100 rpm.

According to an alternate preferred aspect, the rubbing member is cylindrical. Preferably, the rubbing means includes a pair of mutually counter-rotatable cylindrical rubbing members which are spaced apart to create a gap wherein the gloved hand can be inserted for contacting with the rubbing means. Angular velocity of cylindrical rubbing members is preferably between 1-200, preferably 10-150, more preferably between 30-100 rpm.

According to a preferred aspect, the rubbing member includes a contact member in contact with said glove. The contact member is made of flexible or resilient material, so that the rubbing does not cause inconvenience and yet maintains an intimate contact between the gloved hand and the contact member. The contact member is selected from bristles, sponge, a textured rubber surface, or foam.

Preferably, the gloved hand is held steady and in dynamic contact with the contact member as the contact member rubs against the gloved hand.

Any suitable method for measurement of microbial count can be used..

### Preferred Embodiment

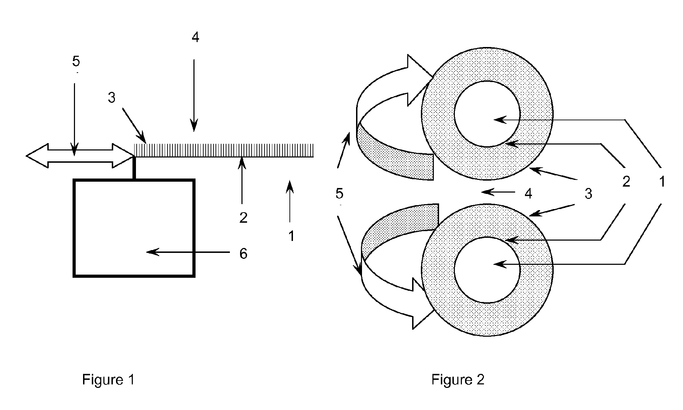

Figure 1 is one of the preferred embodiments of a mechanical rubbing means according to the present invention. The mechanical rubbing means (1) includes a rubbing member (2) which is in form of a flat sheet. The rubbing member in turn includes a contact member (3) having bristles. The gloved hand of a subject is placed at location (4) in contact with the contact member (3). The rubbing member is capable of reciprocating motion in the direction indicated by (5), by using a drive mechanism (6). The reciprocating motion of the contact member (3) with the gloved hand for a predetermined amount of time results into intimate contact of the hand surface with the collection fluid contained within the glove in reproducible and robust manner.

Figure (2) is an alternate preferred embodiment of mechanical rubbing means according to the present invention. The mechanical rubbing means (1) includes a pair of mutually counter-rotatable cylindrical rubbing members (2), which in turn include contact members (3) in form a mat of foam wrapped around the cylindrical rubbing members. The gloved hand of a subject is placed at a location (4) in contact with contact members. The rubbing members are capable of counter-rotating in the direction indicated by (5). The rotary motion of the contact member (3) with the gloved hand for a predetermined amount of time results into intimate contact of the hand surface with the collection fluid contained within the glove in reproducible and robust manner.

### Example

The invention will now be demonstrated with an example. The example is by way of an illustration only and does not limit the scope of the invention in any manner.

### Materials and methods

Glove - Plastic bag

Collection fluid - Butterfield's phosphate buffer

Bacterial count method: Standard plate count method.

In the experiments carried out, for both manual method and method as per invention, samples from 11 panelists were collected and used for the measurements.

The process of collection was as follows.

100 µl of *E*. *coli* suspension of 10⁸ cells/ml was applied to the volunteer's hands following disinfection (with 70% alcohol) to remove the normal flora of the hand. For recovery of bacteria from the volunteer's hands following treatment, the hand was placed in sterile polyethylene bags containing 40 ml of collection fluid (Butterfield phosphate buffer with neutralizers) and secured above the wrists with Velcro bands.

### Method as per the invention:

The volunteers were then asked to place their hands on the paddles of the mechanical rubbing means as per Figure -1. The contact member (3) which were paddles having bristles, in this case, moved forward and backward electronically, maintaining a constant desired speed. The paddles covered the surface of the entire palm. The machine was operated at a speed of 150 rpm for 1 min. The recovery step was repeated with 35 ml of buffer. Samples were pooled and plated onto CY agar

### Manual method:

The gloved hands of the volunteer was gently massaged by the investigator for one minute.

The data on bacterial collection efficiency using manual method and the method of the invention is summarized in Table - 1.

**Table - 1**

| | Manual method | Method of the invention |
|---|---|---|
| Bacteria loading in the collection fluid-Butterfield phosphate buffer | 7.0 log/hand | 7.0 log/hand |
| Bacteria collection efficiency from glove juice | 44% | 66% |
| Mean bacterial count | 6.5 | 6.7 |
| Standard deviation in bacterial count | 0.26 | 0.15 |

The data in Table - 1 indicates that the method as per the invention provides for significantly lower standard deviation in the bacterial count thereby having a much lower variability in the bacterial count.

## Claims

1. An improved glove juice method for measuring microbial count on hands comprising the steps of:
(i) Placing a hand of a subject in a sterile glove, prior to or after pouring a collection fluid in the glove;
(ii) Contacting the gloved hand with a mechanical rubbing means for a predetermined amount of time to intimately contact hand surface with the collection fluid;
(iii) Collecting the glove juice and measuring microbial count in the glove juice.

2. An improved glove juice method as claimed in claim 1 comprising the steps of releasably securing said glove at the wrist of the subject by a securing means to prevent spillage or leakage after step (i) but before step (ii).

3. An improved glove juice method as claimed in claim 1 or claim 2 wherein said rubbing means include at least one rubbing member.

4. An improved glove juice method as claimed in claim 3 wherein said rubbing member is a flat sheet.

5. An improved glove juice method as claimed in claim 4 wherein said sheet is capable of a reciprocal or rotary motion within the plane of said sheet.

6. An improved glove juice method as claimed in claim 3 wherein said rubbing member is cylindrical.

7. An improved glove juice method as claimed in claim 6 wherein said rubbing means includes a pair of mutually counter-rotatable cylindrical rubbing members; said members are spaced apart to create a gap wherein the gloved hand can be inserted for said contacting.

8. An improved glove juice method as claimed in any one of the preceding claims wherein said rubbing member includes a contact member in contact with said glove

9. An improved glove juice method as claimed in claim 8 wherein said contact member is selected from bristles, sponge, or a textured rubber surface.
